(19) Europäisches Patentamt
European Patent Office
Office européen des brevets

(11) **EP 3 782 540 A1**

(12) **EUROPEAN PATENT APPLICATION**

(43) Date of publication:
**24.02.2021 Bulletin 2021/08**

(51) Int Cl.:
***A61B 5/00*** (2006.01)

(21) Application number: **20191701.0**

(22) Date of filing: **19.08.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.08.2019 US 201962888556 P
18.08.2020 US 202016996878**

(71) Applicant: **Vitalchains Corporation
Menlo Park, CA 94025 (US)**

(72) Inventors:
• **Wang, Tao-Wei
115 Taipei City (TW)**
• **Hung, Chih-Wen
115 Taipei City (TW)**
• **Jing, Ming-Chiuan
115 Taipei City (TW)**
• **Lan, Shih-Cheng
115 Taipei City (TW)**

(74) Representative: **Straus, Alexander
2K Patent- und Rechtsanwälte - München
Keltenring 9
82041 Oberhaching (DE)**

(54) **METHOD FOR DETERMINING R PEAKS OF ELECTROCARDIOGRAM**

(57) The present disclosure provides a method for determining R peaks of an electrocardiogram(ECG/EKG). First, an ECG/EKG complex (100) is provided, and then, a maximum peak (MPK) of the ECG/EKG complex (100) is obtained. Following that, a half of a largest voltage (VT1) of the maximum peak (MPK) is defined as a threshold voltage. Later, an R peak number estimating process is performed to obtain an estimated number of all R peaks of the ECG/EKG complex (100) and a plurality of peaks (PK) of the ECG/EKG complex (100) with voltages greater than the threshold voltage and followed by determining whether a number of the peaks (PK) is equal to the estimated number of the all R peaks. When the number of the peaks (PK) is equal to the estimated number of the all R peaks, the peaks (PK) serve as the all R peaks.

FIG. 1

EP 3 782 540 A1

**Description**

Field of the Invention

The present invention relates to a method for determining R peaks of electrocardiogram (ECG/EKG) according to the pre-characterizing clauses of claim 1.

Background of the Invention

Electrocardiogram (ECG/EKG) is mostly used for monitoring electrical activity of the heart. It is considered to be non-invasive and can provide immediate results. Through the ECG/EKG, some heart diseases may be found.
Since various patterns of ECG signals may be obtained in different cardiac diseases, for example, two R waves are generated in right bundle branch block (RBBB) case, and T wave with high amplitude voltage and low slope in ST-segment elevation myocardial infarction (STEMI) case, it is difficult to find or locate the position of the R peaks of the ECG signal. Also, in a vibration environment, the detected ECG signal may be uneven, leading to hardly find or locate the position of the R peaks of the ECG signal.
Some approaches are proposed for finding the position of the R peaks, such as using complex wavelet to calculate multiple resolution feature of ECG signal to determine R peak, using first or second derivative differentiation to acquire the ECG variation and locate the position of the R peak, or using machine learning.
However, the wavelet-based approach needs multiple frequency conversions, resulting in highly complex calculation; the deep machine learning approach needs big data base as training set, and data base with high quality training set is not easy to collect, thereby complicating the approach; and the approach using the first or second derivative differentiation is not easy to accurately detect the R peaks of waveforms with large ripple or large shape variance, leading to affect the accuracy of automated diagnosis. Therefore, providing a simple method for determining the R peaks is a subject in the related field.

Summary of the Invention

This in mind, the present invention aims at providing a method for determining R peaks of electrocardiogram (ECG/EKG), such that the R peaks can be easily and accurately located.
This is achieved by providing the method for determining R peaks of ECG/EKG according to the claims. The dependent claims pertain to corresponding further developments and improvements.
As will be seen more clearly from the detailed description following below, a method for determining R peaks of an electrocardiogram(ECG/EKG) is provided by the present invention. First, an ECG/EKG complex is provided and followed by obtaining a maximum peak of the ECG/EKG complex. Later, a half of a largest voltage of the maximum peak as a threshold voltage is obtained, and then, an R peak number estimating process is performed to obtain an estimated number of all R peaks of the ECG/EKG complex and a plurality of peaks of the ECG/EKG complex with voltages higher than the threshold voltage. Next, whether a number of the plurality of peaks is equal to the estimated number of the all R peaks is determined. When the number of the plurality of peaks is equal to the estimated number of the all R peaks, the plurality of peaks are regarded as the all R peaks.
The present invention provides a method for automatically determining R peaks of ECG/EKG, in which the method utilizes the characteristic of beating cycle of heart that doesn't change suddenly as well as similar adjacent R peaks and includes auto-adjusting the threshold voltage the ECG/EKG complex, so the number of the R peaks can be easily and accurately found, thereby locating the R peaks. Also, the method doesn't need complicated calculation of the wavelet-based approach and is easily implemented in the portable device, also providing power saving function because of simple calculation. Furthermore, the method also doesn't require pre-annotating the ECG/EKG.

Brief Description of the Drawings

In the following, the disclosure is further illustrated by way of example, taking reference to the accompanying drawings. Thereof:

FIG. 1 schematically illustrates a system for determining R peaks of an electrocardiogram (ECG/EKG) according to an embodiment of the present invention;
FIG. 2 schematically illustrates a flowchart of a method for determining R peaks of an ECG/EKG complex according to an embodiment of the present invention;
FIG. 3 schematically illustrates an example of an ECG/EKG complex; and
FIG. 4 schematically illustrates a flowchart of the R peak number estimating process according to the embodiment of the present invention.

Detailed Description

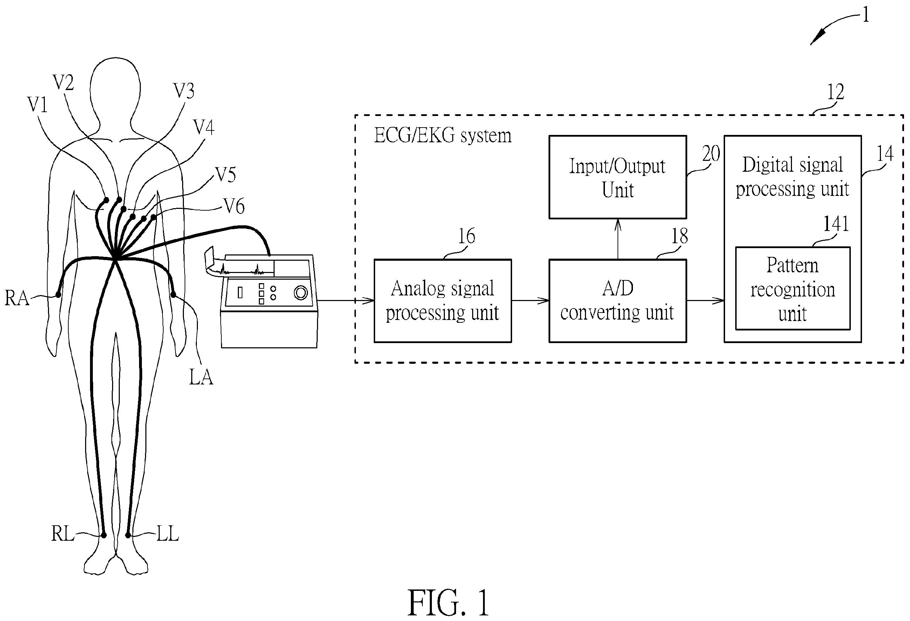

FIG. 1 schematically illustrates a system for determining R peaks of an electrocardiogram (ECG/EKG) according to an embodiment of the present invention, in which the R peaks may be also called R points. The system 1 includes a plurality of ECG/EKG electrodes for detecting and receiving the ECG/EKG signal and an ECG/EKG system 12 for determining the R peaks of ECG/EKG. The ECG/EKG system 12 is operatively coupled to a patient by attaching 10 ECG/EKG electrodes, such as electrodes V1, V2, V3, V4, V5, V6, RA, LA, RL and LL, to the body of the patient, so as to detect signals of 12 leads (I, II, III, aVR, aVL, a VF, V1, V2, V3, V4, V5 and V6) of the ECG/EKG. In the present invention, the ECG/EKG electrodes detect these signals and transmit these signals to the ECG/EKG system 12 for further processing, and thus the R peaks can be determined. The ECG/EKG electrodes may be for example implemented in a wearable device, such as watch, a mobile ECG machine, or other suitable devices.

In this embodiment, the ECG/EKG system 12 may include a digital signal processing unit 14 for determining the R peaks according to the following determining methods. For example, the digital signal processing unit 14 may include a pattern recognition unit 141 for determining PQRST complex waveforms that may include the plural R peaks. One of the PQRST complex waveforms may be one ECG cardiac cycle of the signal of any one of 12 leads, for example one ECG cardiac cycle of lead V2 or lead V3, but not limited thereto. One normal PQRST complex waveform may for example include one P wave representing the depolarization of the atria, one QRS complex representing the depolarization of the ventricles, and one T wave representing repolarization of ventricle, thereby for example including P, Q, R, S, and T points. In some embodiments, the ECG/EKG electrodes may collect analog ECG/EKG signals. In such situation, the ECG/EKG system 1 may further include an analog signal processing unit 16 and an A/D converting unit 18 for processing the analog ECG/EKG signal into a digital ECG/EKG signal for further R peaks searching. In some embodiments, the analog signal processing unit 16 may optionally include filter, amplifier, level shifter or rectifier, but not limited thereto. After the R peaks are determined, the PQRST complex waveforms including the R peaks may be further analyzed by the digital signal processing unit 14 (e.g. the pattern recognition unit 141) for recognizing the pattern of the PQRST complex waveform and extracting its feature (e.g. the J point). Accordingly, doctors, patients, or AI data analytics may be assisted by the processed data to have further clinical decision support.

In some embodiments, the ECG/EKG system 12 may further include an input/output unit 20, in which the input/output unit 20 may for example be a display panel or printer for showing the ECG/EKG to assist doctor to make a diagnosis.

In some embodiments, the ECG/EKG electrodes may collect digital signal and thus the digital ECG/EKG signal is transmitted to the digital signal processing unit 14 directly. The following method for determining R peaks is not limited to be applied to the digital signal processing unit 14 of the above-mentioned system 1.

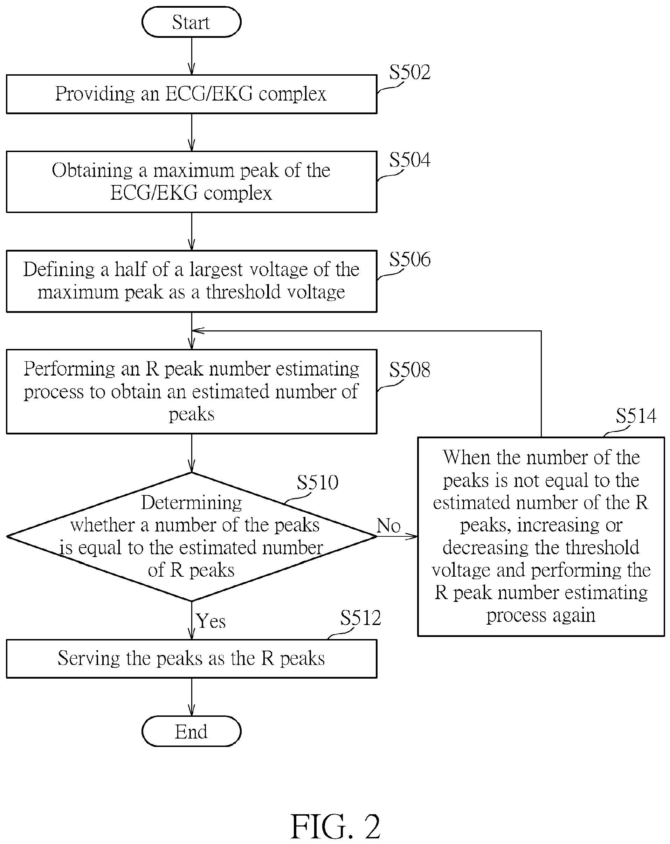

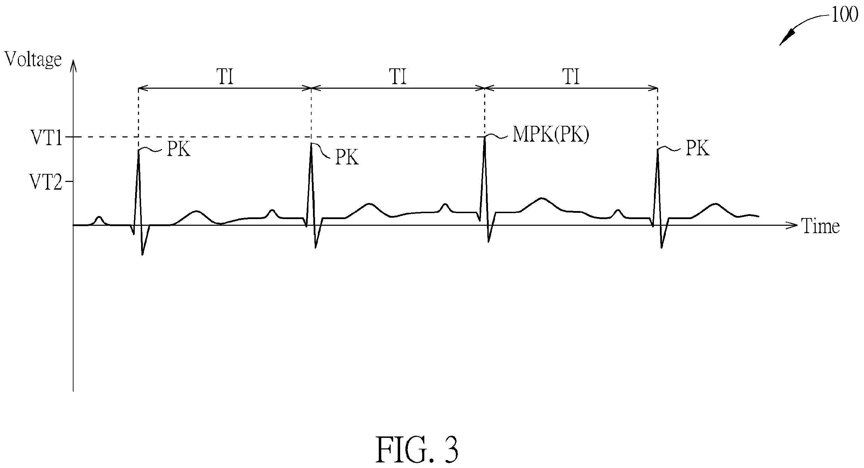

FIG. 2 schematically illustrates a flowchart of a method for determining R peaks of an ECG/EKG complex according to an embodiment of the present invention, and FIG. 3 schematically illustrates an example of an ECG/EKG complex. The method of this embodiment may include steps S502-S514, which are automatically performed by the above system 1 shown in FIG. 1. As shown in FIG. 2, the method starts from step S502. In step S502, the ECG/EKG complex 100 (as shown in FIG. 3) is provided for example by the ECG/EKG electrodes. For example, the ECG/EKG complex 100 may be measured and recorded by utilizing the ECG/EKG electrodes to receive the ECG/EKG signal from a patient for such as about 60 seconds. The ECG/EKG complex 100 may include at least one of signals of 12 leads. In step S504, after the ECG/EKG complex 100 is provided, a maximum peak MPK of the ECG/EKG complex 100 is obtained for example by the digital signal processing unit. For example, the maximum peak MPK can be found by searching one point of the ECG/EKG complex that has a largest voltage VT1.

In step S506, a half of the largest voltage VT1 of the maximum peak MPK (such as an voltage VT2) is calculated and defined as a threshold voltage for example by the digital signal processing unit. In step S508, an R peak number estimating step is performed to obtain an estimated number of all R peaks (N) of the ECG/EKG complex 100 and a plurality of peaks PK of the ECG/EKG complex 100 with voltages greater than the threshold voltage (e.g. the voltage VT2), in which the maximum peak MPK is one of the peaks PK. For example, the peaks PK can be found by searching peak points of the ECG/EKG complex that have the voltages greater than the half of the largest voltage VT1.

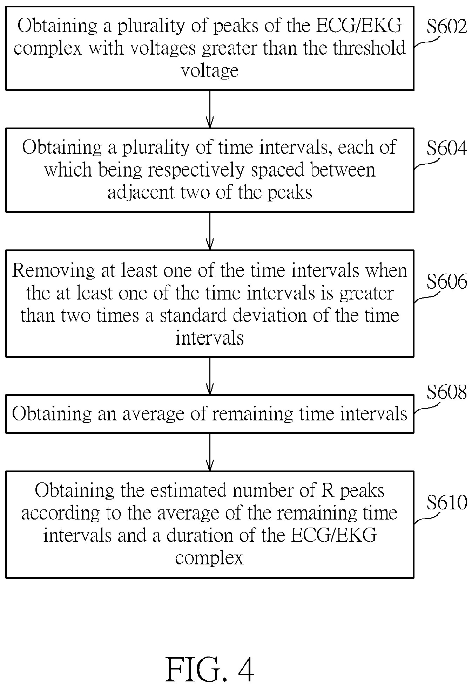

FIG. 4 schematically illustrates a flowchart of the R peak number estimating process according to the second embodiment of the present invention. As shown in FIG. 4, the R peak number estimating process includes steps S602-S610. In step S602, the peaks PK of the ECG/EKG complex 100 with voltages greater than the threshold voltage (e.g. the voltage VT2) are obtained according to the threshold voltage. For example, the peaks PK may be found by using a comparator in the digital signal processing unit to search peak points of the ECG/EKG complex 100 that have the voltages greater than the threshold voltage. In step S604, a plurality of time intervals TI may be calculated and obtained, in which each of the time intervals TI is respectively spaced between adjacent two of the peaks PK. For example, the time interval TI is an interval between time points of the corresponding adjacent peaks PK. Two of the obtained time intervals TI may be the same or different. In step S606, when at least one of the time intervals TI is greater than two times a standard deviation of the time intervals TI, the at least one of the time intervals TI is removed to ensure that the data of remaining time intervals TI can be close to time interval between adjacent two of the desired R peaks. The standard deviation is a measure of the amount of variation or dispersion of the time intervals TI. Specifically, the standard deviation may be calculated by the following formula (1).

$$sd = \sqrt{\frac{1}{n-1} \sum_{i=1}^{n} \left( x_i - \bar{x} \right)^2} \qquad (1),$$

where sd is the standard deviation, n is the number of the peaks, $x_1$, $x_2$,..., $x_n$ are the voltages of the peaks PK, and $\bar{x}$ is the average of the voltages of the peaks PK. In step S608, an average of the remaining time intervals TI is calculated and obtained. In step S610, the estimated number of R peaks (N) is evaluated and obtained according to the average of the remaining time intervals TI and the duration of the ECG/EKG complex 100. For example, the estimated number of R peaks (N) is obtained by dividing the duration by the average of the remaining time intervals TI. The average may be for example a mean, a median or a mode of the remaining time intervals TI, but not limited thereto.

In step S510, after the estimated number of the R peaks (N) and the number of the peaks PK (n) are obtained, whether the number of the peaks PK (n) is equal to the estimated number of the R peaks (N) is determined by the digital signal processing unit. When the number of the peaks PK (n) is equal to the estimated number of the R peaks (N), the method proceeds to step S512, in which the peaks PK are regarded as the R peaks. Accordingly, all the R peaks can be found, and the method is ended.

When the number of the peaks PK (n) is not equal to the estimated number of the R peaks (N), the method proceeds to step S514 and includes increasing or decreasing the threshold voltage by a predetermined voltage to be another threshold voltage. For example, the threshold voltage may be increased or decreased by the digital signal processing unit. The predetermined voltage may be preset or stored in the digital signal processing unit. Then, the method returns to step S508, and the R peak number estimating process is performed again to find the peaks PK based on the another threshold voltage until the estimated number of the R peaks is equal to the number of the found peaks PK. Specifically, when the number of the peaks PK (n) is less than the estimated number of the R peaks (N), the threshold voltage is decreased to another threshold voltage, and the R peak number estimating process is performed again. On the other hands, when the number of the peaks PK (n) is greater than the estimated number of the R peaks (N), the threshold voltage is increased to another threshold voltage, and the R peak number estimating process is performed again.

It is noted that since beating cycle of heart doesn't change suddenly, the time intervals TI between the R peaks are almost the same. Also, because voltages of the adjacent R peaks are similar and large enough, through adjusting the threshold voltage, the peaks can be found, and then, the time intervals TI can be estimated. For this reason, through the loop of adjusting the threshold voltage, the number of the R peaks can be easily and accurately found, thereby locating the R peaks.

As mentioned above, in the method for determining R peaks of the present invention, since the calculation for determining the R peaks is simple and easy, no complicated calculation of the wavelet-based approach is required, and thus, the method is easily implemented in the portable device. Also, the method may further achieve energy saving effect because of simple calculation and/or may not require pre-annotating the ECG/EKG.

**Claims**

1.    A method for determining R peaks of an electrocardiogram(ECG/EKG), **characterized by:**

   providing an ECG/EKG complex (100);
   obtaining a maximum peak (MPK) of the ECG/EKG complex (100);
   defining a half of a largest voltage (VT1) of the maximum peak (MPK) as a threshold voltage;
   performing an R peak number estimating process to obtain an estimated number of all R peaks of the ECG/EKG complex (100) and a plurality of peaks (PK) of the ECG/EKG complex (100) with voltages higher than the threshold voltage;
   determining whether a number of the plurality of peaks (PK) is equal to the estimated number of the all R peaks; and
   when the number of the plurality of peaks (PK) is equal to the estimated number of the all R peaks, the plurality of peaks (PK) are regarded as the all R peaks.

2. The method for determining R peaks of the ECG/EKG according to claim 1, **characterized in that** when the number of the plurality of peaks (PK) is less than the estimated number of the all R peaks, the method further comprises decreasing the threshold voltage and performing the R peak number estimating process again.

3. The method for determining R peaks of the ECG/EKG according to claim 1, **characterized in that** when the number of the plurality of peaks (PK) is greater than the estimated number of the all R peaks , increasing the threshold voltage and performing the R peak number estimating process again.

4. The method for determining R peaks of the ECG/EKG according to claim 1, **characterized in that** the R peak number estimating process comprises:

obtaining the plurality of peaks (PK) of the ECG/EKG complex (100) with the voltages greater than the threshold voltage according to the threshold voltage;
obtaining a plurality of time intervals (TI), wherein one of the plurality of time intervals (TI) is spaced between adjacent two of the plurality of peaks (PK);
removing at least one of the plurality of time intervals (TI) when the at least one of the plurality of time intervals (TI) is greater than two times a standard deviation of the plurality of time intervals (TI);
obtaining an average of remaining time intervals (TI) of the plurality of time intervals (TI); and
obtaining the estimated number of the all R peaks according to the average of the remaining time intervals (TI) and a duration of the ECG/EKG complex (100).

5. The method for determining R peaks of the ECG/EKG according to claim 4, **characterized in that** the average is a mean, a median or a mode of the remaining time intervals (TI).

6. The method for determining R peaks of the ECG/EKG according to claim 4, **characterized in that** the estimated number of the all R peaks is obtained by dividing the duration of the ECG/EKG complex (100) by the average of the remaining time intervals (TI).

FIG. 1

Start

Providing an ECG/EKG complex    S502

Obtaining a maximum peak of the ECG/EKG complex    S504

Defining a half of a largest voltage of the maximum peak as a threshold voltage    S506

Performing an R peak number estimating process to obtain an estimated number of peaks    S508

Determining whether a number of the peaks is equal to the estimated number of R peaks    S510

No →

When the number of the peaks is not equal to the estimated number of the R peaks, increasing or decreasing the threshold voltage and performing the R peak number estimating process again    S514

Yes

Serving the peaks as the R peaks    S512

End

FIG. 2

FIG. 3

```
┌─────────────────────────────────────────────┐
│ Obtaining a plurality of peaks of the ECG/EKG │   S602
│ complex with voltages greater than the threshold │
│                    voltage                    │
└─────────────────────────────────────────────┘
                       │
                       ▼
┌─────────────────────────────────────────────┐
│ Obtaining a plurality of time intervals, each of │  S604
│   which being respectively spaced between     │
│          adjacent two of the peaks            │
└─────────────────────────────────────────────┘
                       │
                       ▼
┌─────────────────────────────────────────────┐
│ Removing at least one of the time intervals when │
│  the at least one of the time intervals is greater │  S606
│  than two times a standard deviation of the time │
│                    intervals                  │
└─────────────────────────────────────────────┘
                       │
                       ▼                          S608
┌─────────────────────────────────────────────┐
│ Obtaining an average of remaining time intervals │
└─────────────────────────────────────────────┘
                       │
                       ▼
┌─────────────────────────────────────────────┐
│   Obtaining the estimated number of R peaks   │
│  according to the average of the remaining time │  S610
│    intervals and a duration of the ECG/EKG    │
│                    complex                    │
└─────────────────────────────────────────────┘
```

# FIG. 4

9

Europäisches
Patentamt

European
Patent Office

Office européen
des brevets

# EUROPEAN SEARCH REPORT

Application Number

EP 20 19 1701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 309 917 A (WANG XIANG [US] ET AL) 10 May 1994 (1994-05-10) * abstract * * column 8, line 18 - line 60 * ----- | 1-6 | INV. A61B5/0456 |
| X | XUANYU LU ET AL: "QRS Detection Based on Improved Adaptive Threshold", JOURNAL OF HEALTHCARE ENGINEERING, vol. 2018, 15 March 2018 (2018-03-15), pages 1-8, XP055609591, Brentwood ISSN: 2040-2295, DOI: 10.1155/2018/5694595 * abstract * * section 3.2 * * section 3.3 * ----- | 1-6 | |

TECHNICAL FIELDS
SEARCHED (IPC)

A61B

The present search report has been drawn up for all claims

| Place of search | Date of completion of the search | Examiner |
|---|---|---|
| The Hague | 30 November 2020 | Gooding Arango, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## EP 3 782 540 A1

**ANNEX TO THE EUROPEAN SEARCH REPORT
ON EUROPEAN PATENT APPLICATION NO.**

EP 20 19 1701

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2020

| Patent document cited in search report | | Publication date | Patent family member(s) | | Publication date |
|---|---|---|---|---|---|
| US 5309917 | A | 10-05-1994 | AT | 209873 T | 15-12-2001 |
| | | | CA | 2118930 A1 | 18-03-1993 |
| | | | DE | 69232267 T2 | 24-10-2002 |
| | | | EP | 0606301 A1 | 20-07-1994 |
| | | | JP | H07503380 A | 13-04-1995 |
| | | | US | 5309917 A | 10-05-1994 |
| | | | US | 5443073 A | 22-08-1995 |
| | | | WO | 9304627 A1 | 18-03-1993 |

EPO FORM P0459

For more details about this annex : see Official Journal of the European Patent Office, No. 12/82